# FASCICULE DE BREVET EUROPEEN

(11) **EP 1 590 670 B1**
(45) Date de publication et mention de la délivrance du brevet: **21.05.2008**
(21) Numéro de dépôt: 04709254.9
(22) Date de dépôt: 09.02.2004
(51) Int. Cl.: G01N 33/569

(54) **TEST DE DIAGNOSTIC IN VITRO D'INFECTIONS ENTEROVIRALES**
IN VITRO DIAGNOSTISCHER TEST VON ENTEROVIRUSINFEKTIONEN
IN VITRO DIAGNOSTIC TEST FOR ENTEROVIRAL INFECTIONS

(30) Priorité: 07.02.2003 FR 0301454
(43) Date de publication de la demande: 02.11.2005
(73) Titulaire: Université de Lille 2 Droit et Santé, 59800 Lille (FR); Centre Hospitalier Regional Universitaire de Lille, 59037 Lille Cédex (FR)
(72) Inventeur: CHEHADEH, Wassim, F-59000 Lille (FR); BOUZIDI, Ahmed, F-59112 Annoeulin (FR); HOBER, Didier, F-59133 Camphin en Carembault (FR)
(74) Mandataire: Lepeudry-Gautherat, Thérèse
(86) Numéro de dépôt international: PCT/FR2004/000287
(87) Numéro de publication internationale: WO 2004/072644

(56) Documents cités:
- EP-A- 0 434 992
- DE-A- 19 846 271
- HOBER DIDIER ET AL: "Circulating and cell-bound antibodies increase coxsackievirus B4-induced production of IFN-alpha by peripheral blood mononuclear cells from patients with type 1 diabetes." JOURNAL OF GENERAL VIROLOGY, vol. 83, no. 9, septembre 2002 (2002-09), pages 2169-2176, XP002243774 September, 2002 ISSN: 0022-1317
- MULDERS MICK N ET AL: "Molecular epidemiology of coxsackievirus B4 and disclosure of the correct VP1/2Apro cleavage site: Evidence for high genomic diversity and long-term endemicity of distinct genotypes" JOURNAL OF GENERAL VIROLOGY, vol. 81, no. 3, mars 2000 (2000-03), pages 803-812, XP002287097 ISSN: 0022-1317
- GIRN JASKAMAL ET AL: "Enhancement of coxsackievirus B3 infection by antibody to a different coxsackievirus strain." JOURNAL OF GENERAL VIROLOGY, vol. 83, no. 2, février 2002 (2002-02), pages 351-358, XP002243776 February, 2002 ISSN: 0022-1317
- KUBO HIDEYUKI ET AL: 'Molecular classification of enteroviruses not identified by neutralization tests' EMERGING INFECTIOUS DISEASES vol. 8, no. 3, Mars 2002, pages 298 - 304, XP007902644

### SEQUENCE LISTING

<110> CENTRE HOSPITALIER REGIONAL UNIVERSITAIRE DE LILLE UNIVERSITE DE LILLE 2 DROIT ET SANTE
<120> Test de diagnostic in vitro d'infections entérovirales
<130> 10476/5PCT
<150> FR0301454
   <151> 2003-02-07
<160> 5
<170> PatentIn version 3.1
<210> 1
   <211> 20
   <212> PRT
   <213> Coxsackievirus B
<400> 1
   Met Gly Ala Gln Val Ser Thr Gln Lys Thr Gly Ala His Glu Thr Ser
   1               5                   10                  15
   Leu Ser Ala Ser
               20
<210> 2
   <211> 20
   <212> PRT
   <213> Coxsackievirus B
<400> 2
   Gly Ala His Glu Thr Ser Leu Ser Ala Ser Gly Asn Ser Ile Ile His
   1               5                   10                  15
   Tyr Thr Asn Ile
               20
<210> 3
   <211> 20
   <212> PRT
   <213> coxsackievirus B
<400> 3
   Gly Asn Ser Ile Ile His Tyr Thr Asn Ile Asn Tyr Tyr Lys Asp Ala
   1               5                   10                  15
   Ala Ser Asn Ser
               20
<210> 4
   <211> 30
   <212> PRT
   <213> Coxsackievirus B
<400> 4
   Asn Tyr Tyr Lys Asp Ala Ala Ser Asn Ser Ala Asn Arg Gln Asp Phe
   1               5                   10                  15
   Thr Gln Asp Pro Ser Lys Phe Thr Glu Pro Val Lys Asp Val
               20                  25                  30
<210> 5
   <211> 19
   <212> PRT
   <213> Coxsackievirus B
<400> 5
   Ser Lys Phe Thr Glu Pro Val Lys Asp Val Met Ile Lys Ser Leu Pro
   1               5                   10                  15
   Ala Leu Asn

## Description

L'invention concerne un test de diagnostic in vitro d'entérovirus basé sur la révélation d'une réaction immunologique de type reconnaissance antigène-anticorps.

Les entérovirus forment le genre Entérovirus au sein de la famille des *Picornaviridae.* Il s'agit de virus de 25 à 30 nm de diamètre, non enveloppés, à symétrie icosaédrique et à ARN (acide ribonucléique) monocaténaire linéaire, non fragmenté et positif.

Leur absence d'enveloppe leur confère une résistance aux agents physico-chimiques et une stabilité entre pH 3 et 10. Par contre, les entérovirus sont inactivés par la chaleur au-delà de 45°C (et même 50°C en présence de cations divalents) et par les désinfectants et antiseptiques majeurs (povidone iodée, hypochlorite de sodium, aldéhydes).

Il existe 64 sérotypes d'entérovirus : 3 poliovirus, 23 coxsackievirus A (CVA), 6 coxsackievirus B (CVB), 28 échovirus (EV) et 4 entérovirus non classés.

Plus de 80% du génome des entérovirus d'environ 7500 nucléotides est constitué d'un cadre de lecture unique flanqué de deux régions non codantes en 5' et 3'. La volumineuse protéine codée par ce cadre de lecture est clivée en 4 protéines matures structurales VP1, VP2, VP3 et VP4 (VP=viral protein) et en protéines non structurales comme des protéases et l'ARN polymérase virale. Peu de génomes d'entérovirus animaux ont été séquencés. Cependant, ceux qui l'ont été montrent une très grande homologie avec les entérovirus humains.

Les entérovirus sont l'objet d'une grande variabilité génétique due aux erreurs de transcription de l'ARN polymérase virale sources de mutations ponctuelles et aux recombinaisons entre génomes de virus différents. Cette variabilité contribue à la diversité des tropismes tissulaires et des spectres pathologiques des entérovirus.

Les entérovirus ont un mode de transmission fécal-oral par contacts entre deux individus de la même espèce ou par le biais d'eaux ou d'aliments contaminés. Certains sérotypes ont une transmission respiratoire ou cutanéo-muqueuse. Les entérovirus qui pénètrent par voie digestive se multiplient d'abord dans l'intestin (d'où le terme *entérovirus*), avant de diffuser, par voie hématogène, vers des organes cibles (système nerveux central, muscles striés, peau...). Les infections inapparentes constituent la grande majorité des entéroviroses. Parmi les formes symptomatiques, on distingue les infections aiguës et les infections persistantes.

Les infections aiguës chez l'homme sont très polymorphes. Les entérovirus sont les agents infectieux les plus fréquents responsables d'infections du système nerveux central (méningites lymphocytaires, méningo-encéphalites, paralysies de type poliomyélite). Ils participent à de nombreuses autres pathologies : infections respiratoires (rhinites, bronchites, bronchiolites, pneumonies), infections cardiaques (péricardites, myocardites), myosites, éruptions maculo-papuleuses ou purpuriques, syndromes fébriles et, plus rarement, hépatites, néphrites, orchites ou arthrites. Des manifestations cliniques sont très spécifiques des entérovirus et même de certains sérotypes : pleurodynie ou maladie de Bornholm (CVB) qui correspond à une sorte de grippe hyperalgique, éruptions vésiculeuses de type herpangine ou syndrome pied-main-bouche (CVA, CVB, entérovirus 70), conjonctivite hémorragique (CVA-24, entérovirus 70).

Chez les animaux, les entérovirus touchent les bovins, les porcins et les volailles. La plupart de ces infections entérovirales sont inapparentes et seuls les entérovirus porcins et aviaires causent des maladies d'importance économique. Certaines souches d'entérovirus porcins sont responsables de la polioencéphalomyélite porcine. Le SVDV (swine vesicular disease virus) cause une maladie vésiculaire des porcins. En général, la maladie en elle-même n'est pas sévère et la plupart des animaux en réchappent.

Les pathologies humaines chroniques associées aux entérovirus sont au moins au nombre de quatre : méningo-encéphalites chroniques, syndrome post-poliomyélitique, affections cardiaques et diabète insulino-dépendant. En effet, il existe de forts arguments en faveur de l'implication des coxsackievirus B dans le diabète mellitus insulino-dépendant (DID) ou diabète de type 1. Plusieurs auteurs ont détecté la présence d'ARN entéroviral de forte homologie avec CVB dans le sang périphérique de patients DID au début des manifestations cliniques de la maladie (Clements et al., 1995; Andreoletti et al., 1997; Nairn et al., 1999; Lonnrot et al., 2000). Récemment la Demanderesse a montré que des taux élevés dans le plasma d'interféron α (IFNα) sont corrélés dans 50% des cas avec la présence de séquences entérovirales, en particulier CVB3 et CVB4, dans le sang circulant d'adultes et d'enfants atteints de diabète de type 1 (Chehadeh et al., 2000). La Demanderesse a également démontré que CVB4, par des interactions avec des anticorps IgG circulants ou associés à des cellules, peut fortement induire la production d'IFNα par des cellules mononucléées du sang périphérique (PBMC) de patients DID (Hober et al., J. Gen. Virol., vol. 83, n°9, septembre 2002). La production d'interféron α est un marqueur de l'infection virale. CVB4 induit peu cette production sauf quand il est mis en présence d'anticorps dits "facilitants" qui facilitent l'infection virale. CVB4 peut donc infecter des monocytes, en majorité CD14+, par un mécanisme anticorps-dépendant par des interactions entre le virus, des anticorps antiviraux et des récepteurs spécifiques à la surface des cellules (CAR, Fcy RII, Fcy RIII) qui résultent en la production d'IFNα. Cette synthèse d'IFNα induite par des IgG anti-CVB4 reflète l'entrée de CVB4 dans les monocytes mais pas la réplication virale et requiert la présence d'ARN de CVB4 dans les cellules. Si l'on bloque la production d'IFNα induite par ces IgG anti-CVB4, des particules virales produites par les PBMC peuvent être détectées (Chehadeh et al., J. Gen. Virol., vol.82, n°8, août 2001). De plus, l'activité inductrice d'IFNα de plasma de patients IDDM préincubé avec CVB4 avant d'être apporté à des PBMC isolées de sujets sains est élevée si on la compare à celle de plasma de sujets sains (Hober et al., J. Gen. Virol., vol. 83, n°9, septembre 2002). Les patients IDDM ont une prévalence plus élevée de ces anticorps anti-CVB dits "facilitants" qui augmentent la synthèse d'IFNα induite par CVB par rapport aux contrôles. Les patients atteints d'infections entérovirales portent donc dans leur plasma, en plus des anticorps neutralisants connus jusqu'à présent qui "immobilisent" le virus et qui sont dans la majorité des cas dirigés contre des épitopes de la protéine structurale de surface VP1, des anticorps "facilitants" qui favorisent l'infection virale.

Les outils diagnostiques utilisés jusqu'à présent dans le diagnostic des entéroviroses sont des moyens directs : culture cellulaire, inoculation au souriceau nouveau-né, amplification génomique en utilisant des amorces dans la région 5' non codante du génome et des moyens indirects : séroneutralisation et techniques immuno-enzymatiques.

La culture cellulaire et la séroneutralisation ne sont pas applicables à tous les coxsackievirus A, les sérotypes A1, A19 et A22 ne sont pas cultivables. En pratique, la plupart des CVA ne sont pas facilement cultivables sauf CVA9.

L'inoculation au souriceau nouveau-né est une technique lourde et longue qui permet de diagnostiquer une infection à coxsackievirus et permet de différencier les CVA (paralysies flasques) des CVB (paralysies spastiques).

Les techniques de biologie moléculaire comme l'amplification génomique par PCR (Polymerase Chain Reaction) ont permis de détecter les entérovirus en faible quantité en choisissant des amorces dans les régions très conservées. Cependant, elles ne permettent pas de détecter toutes les infections entérovirales, en particulier si l'infection est localisée et le taux de réplication du virus faible, ni de différencier tel ou tel sérotype. De même, les techniques immunoenzymatiques différencient tout au plus les groupes et celles-ci, basées sur la détection des anticorps neutralisants, se sont heurtées à l'absence d'un antigène commun parmi les entérovirus.

La demande de brevet européen EP 0434992 (Max Planck Gesellschaft) décrit un test de diagnostic in vitro des Entérovirus, basé sur la révélation d'une réaction immunologique de type reconnaissance antigène-anticorps entre la protéine VP1 du coxsackievirus B3 ou la protéine de fusion VP4/2/3 des protéines VP4, VP2 et VP3 du coxsackievirus B3, et les anticorps dirigés contre ces protéines. Le test diagnostic divulgué dans EP 0434992 permet de diagnostiquer la présence d'un Entérovirus sans toutefois permettre d'en identifier le sérotype.

Kubo et al. (Emerging Infectious Diseases, vol. 8, no. 3, mars 2002, pages 298-304) divulgue qu'une analyse phylogénétique basée sur la comparaison des séquences nucléotidiques de la protéine VP4 se révèle plus performante, pour réaliser le sérotypage d'Entérovirus, que les tests classiques de neutralisation reposant sur des antisérums possédant une spécificité pour certains sérotypes d'entérovirus.

Il n'existe donc pas parmi ces tests une méthode de détection très fine à l'échelon du sérotype ou des variants (différenciation entre souches sauvages et vaccinales) ni de méthode de quantification de la charge virale chez des individus infectés.

Pour combler ce manque, la Demanderesse a développé un test de diagnostic in vitro des entérovirus spécifique et quantitatif. Ce test repose sur l'existence d'antigènes induisant des anticorps "facilitant" l'infection virale et pas d'anticorps neutralisants.

En effet, la Demanderesse a identifié la protéine virale présentant le ou les épitopes reconnus par les anticorps "facilitants" dans le cas d'une infection aux coxsackievirus B3 et B4. Cette protéine est la protéine structurale interne VP4 et la démonstration en a été faite comme suit :
a) tout d'abord, les virus CVB3 et CVB4 E2 ont été cultivés et purifiés
b) ensuite, les protéines VP4 d'une part et l'antigène H (également appelé AEC pour "artificial empty capsids") d'autre part ont été isolés et purifiés à partir des virus CVB3 et CVB4 E2 dissociés
c) enfin, il a été démontré que la protéine VP4 inhibe l'augmentation de la production d'IFNα induite par le couple CVB/plasma. En effet, quand VP4 est préincubée avec du plasma avant d'ajouter CVB, VP4 fixe les anticorps anti-VP4 et il reste moins d'anticorps anti-VP4 pour fixer CVB et faciliter l'infection de PBMC in vitro et l'augmentation de la production d'IFNα par celles-ci. Ceci prouve que les anticorps "facilitants" sont les anticorps anti-VP4.

Par contre, il n'y a pas de réaction croisée entre les anticorps anti-VP4_{CVB3} et anti-VP4_{CVB4}. Quand VP4_{CVB4} a été préincubée avec du plasma avant d'ajouter CVB3, le niveau d'IFNα induit par CVB3 dans des cultures de PBMC est resté inchangé. De même, la protéine VP4 dissociée de CVB3 n'affecte pas le niveau d'IFNα induit par CVB4. Les épitopes présentés par les protéines VP4 de ces deux sérotypes sont donc suffisamment différents pour que les anticorps "facilitants" soient spécifiques de tel ou tel sérotype.

Ce résultat a été vérifié avec les autres sérotypes de CVB, CVB1 à CVB5. Les anticorps anti-VP4_{CVB4E2} ne reconnaissent pas les virus CVB d'un autre sérotype (Exemple 3).

On appelle "anticorps facilitants" des anticorps non neutralisants, qui facilitent l'infection virale et qui sont spécifiques du sérotype du virus dont leur antigène est issu.

La Demanderesse a tiré parti de cette démonstration pour mettre au point un test de diagnostic in vitro d'entérovirus basé sur la révélation d'une réaction immunologique de type reconnaissance antigène-anticorps caractérisé en ce qu'il met en oeuvre des antigènes ou des épitopes de ceux-ci qui n'induisent pas d'anticorps antiviraux neutralisants mais qui induisent des anticorps "facilitants" qui augmentent l'infection virale.

La présente invention a pour objet un test de diagnostic in vitro d'entérovirus basé sur la révélation d'une réaction immunologique de type reconnaissance antigène-anticorps caractérisé en ce que :
- l'antigène mis en oeuvre ou des épitopes de celui-ci, sont ceux qui n'induisent pas d'anticorps antiviraux neutralisants mais qui induisent des anticorps "facilitants" qui augmentent l'infection virale, et
- ledit antigène induisant des anticorps antiviraux « facilitants » est la protéine virale interne structurale VP4, ou un fragment de celle-ci.

Dans un mode de réalisation particulier, la présente invention a donc pour objet un test de diagnostic d'entérovirus caractérisé en ce que soit l'antigène mis en oeuvre ou des épitopes de celui-ci sont fixés sur un support pour la détection des anticorps "facilitants" correspondants, soit les anticorps "facilitants" sont fixés sur un support pour la détection de l'antigène mis en oeuvre ou des épitopes de celui-ci correspondants. Ce support est de type plaque à puits multiples pour microtitration ou "puce" ou tout autre support. On entend par "puce" un support miniaturisé plat, en matériau solide organique ou inorganique, de type verre, silicium ou polymères synthétiques, sur lequel sont liés de manière covalente ou non des polypeptides.

Quand le test selon l'invention consiste en la détection des anticorps "facilitants" par l'antigène mis en oeuvre ou des épitopes de celui-ci correspondants fixés sur un support, la réaction est révélée par un anticorps secondaire anti-espèce marqué (par exemple anti-humain).

Quand le test selon l'invention consiste en la détection de l'antigène mis en oeuvre ou des épitopes de celui-ci par les anticorps "facilitants" correspondants fixés sur un support, la réaction est révélée par un anticorps anti-viral marqué ou par un anticorps anti-viral (par exemple humain) puis un anticorps secondaire anti-espèce marqué (par exemple anti-humain).

Le marqueur des anticorps secondaires est de préférence une enzyme comme la HRP ("horseradish peroxydase") qui montre une réaction colorée ou luminescente avec son substrat, un radioisotope, un composé fluorescent, un composé chimioluminescent, un composé bioluminescent ou un chélate de métaux.

L'antigène induisant des anticorps antiviraux "facilitants" est la protéine virale interne VP4, pleine longueur ou un fragment de celle-ci. Cette protéine peut être purifiée à partir du virus, peut être une protéine recombinante présentant les mêmes propriétés immunogènes ou peut être synthétisée chimiquement et présenter les mêmes propriétés immunogènes.

De manière particulièrement préférée, l'antigène induisant des anticorps antiviraux "facilitants" utilisé dans le test de diagnostic selon l'invention est un fragment peptidique de la protéine VP4 choisi parmi les peptides de séquence SEQ ID N°1, SEQ ID N°2, SEQ ID N°3, SEQ ID N°4, SEQ ID N°5, de préférence le peptide de séquence SEQ ID N°2.

Ce test permet en particulier de diagnostiquer une infection par les coxsackievirus de type A ou B

Ce test comprend les étapes suivantes:
a- immobilisation des anticorps "facilitants" ou de la protéine virale induisant des anticorps "facilitants" ou d'un fragment de celle-ci sur un support
b- immobilisation d'anticorps contrôles, de protéines contrôles ou d'un fragment de celles-ci sur un support
c- lavages avec une solution saline tamponnée additionnée ou non d'un détergent à faible concentration
d- saturation de la surface de support non recouverte par une solution tamponnée de protéines irrelevantes
e- lavages avec une solution saline tamponnée additionnée ou non d'un détergent à faible concentration
f- application des échantillons à étudier à différentes dilutions dans du tampon de saturation
g- lavages avec une solution saline tamponnée additionnée ou non d'un détergent à faible concentration
h- amplification de la réponse par application d'anticorps marqués
i- lavages avec une solution saline tamponnée additionnée ou non d'un détergent à faible concentration
j- lecture de l'intensité du marquage

Le test de l'invention peut en particulier être mis en oeuvre à l'aide d'un coffret ou d'un kit qui comprend :
- la protéine virale interne structurale VP4 ou un fragment de celle-ci ou les anticorps "facilitants" correspondants,
- les réactifs pour la constitution du milieu propice à la réalisation de la réaction antigène-anticorps,
- les réactifs permettant la détection du complexe formé.

Selon l'invention le test peut notamment être appliqué au diagnostic d'une infection à CVB3 ou CVB4 ou tout autre CVB par détection et dosage des anticorps anti-VP4 en les piégeant par la protéine purifiée VP4 fixée à un support. En effet, on a mis en évidence l'existence d'une relation dose-réponse entre la quantité de protéine VP4 préincubée avec le plasma et le niveau de production d'IFNα. Ainsi, la protéine VP4 préincubée avec le plasma piège les anticorps anti-VP4 du plasma. Plus on augmente la quantité de protéine VP4 dans ce test, plus on diminue la quantité d'anticorps anti-VP4 "facilitants" libres pour reconnaître CVB, plus la production d'IFNα est faible. On a de plus mis en évidence l'existence d'une corrélation dose-dépendante entre la quantité d'anticorps anti-VP4 préincubés avec CVB3 ou CVB4 avant d'être ajoutés à des PBMC et le niveau de production d'IFNα. Aucune production d'IFNα n'est détectée en présence de plasma appauvri en anticorps anti-VP4 ou d'anticorps irrelevants.

Le test objet de l'invention, en permettant de doser les anticorps "facilitants" ou la protéine virale portant le ou les épitopes reconnus par ces anticorps, permet de mesurer la réponse des cellules à l'infection virale, comme par exemple la production d'IFNα.

Le test décrit ci-dessus peut être réalisé avec d'autres protéines VP4 d'autres entérovirus pour effectuer un criblage de l'infection par différents virus.

Ce test peut être appliqué dans le cas de maladie chronique associée à une infection par un entérovirus pour établir la valeur prédictive du déclenchement de la maladie. En particulier, ce test peut être utilisé pour prédire le déclenchement chez des patients prédiabétiques d'un diabète de type 1 associé à une infection par CVB.

Ce test peut également être utilisé pour corréler la quantité d'anticorps facilitants détectés et le stade de la maladie. En particulier, ce test peut permettre d'évaluer le stade d'un diabète de type 1 associé à une infection par CVB.

On peut utiliser le test de diagnostic selon l'invention pour déterminer le sérotype de l'entérovirus responsable d'une infection aigüe humaine ou animale. En effet, les anticorps "facilitants" et leur antigène sont spécifiques d'un sérotype entéroviral donné et on peut multiplier le test selon l'invention par autant de sérotypes. Pour ce faire, on peut utiliser autant de supports que de sérotypes ou fixer sur un support miniaturisé de type "puce", pour chaque sérotype, des anticorps "facilitants" ou l'antigène correspondant.

On peut également, de cette façon, utiliser le test de diagnostic selon l'invention pour déterminer la répartition par sérotype des infections entérovirales dans une population donnée humaine ou animale.

Dans une forme de réalisation supplémentaire de l'invention, on peut utiliser le test de diagnostic selon l'invention pour déterminer la cible virale d'un agent antiviral. En effet, on peut détecter et doser l'antigène induisant des anticorps "facilitants", pour chaque sérotype viral, à l'aide du test de diagnostic selon l'invention, en fixant sur un ou plusieurs supports les anticorps "facilitants" correspondants. On peut donc mesurer l'infectiosité de cellules in vitro d'un virus étudié cultivable en absence ou en présence de concentrations plus ou moins importantes d'agent antiviral. On peut faire de même pour plusieurs sérotypes et déterminer la cible de l'agent antiviral. Dans cette forme de réalisation, le test de diagnostic selon l'invention permet de remplacer des mesures longues et fastidieuses de mort cellulaire in vitro en fonction du titre viral et de la concentration en agent antiviral.

Les exemples suivants illustrent l'invention sans toutefois en limiter la portée.

### Légende des figures:

Figure 1 : effet de la protéine VP4 ou de l'antigène H sur la production d'IFNα induite par CVB/plasma de 5 sujets sains
Figure 1a : diminution dose-dépendante de la production d'IFNα induite par CVB3/plasma en présence de la protéine VP4_{CVB3}
Figure 1b : augmentation dose-dépendante de la production d'IFNα induite par CVB3/plasma en présence d'antigène H
Figure 1c : diminution dose-dépendante de la production d'IFNα induite par CVB4/plasma en présence de la protéine VP4_{CVB4}
Figure 1d : augmentation dose-dépendante de la production d'IFNα induite par CVB4/plasma en présence d'antigène H
Figure 2 : comparaison de la moyenne des valeurs indice d'anticorps anti-VP4 des patients DID par rapport à celle des sujets sains
Figure 3 : Moyenne de production d'IFN-alpha par les PBMC après incubation des différents sérotypes de CVB avec le plasma de donneurs sains.
Figure 4 : Evaluation de la spécificité des anticorps activateurs en mesurant par méthode DELFIA la production d'IFNα lors de l'infection des PBMC avec les différents sérotypes du virus en présence de plasma issu de donneur sain (noir), ou en présence de plasma pré-incubé avec VP4 de CVB4E2 (hachuré).
Figure 5 : Production d'IFNα lors de l'infection des PBMC par CVB4E2 ou CVB3 en présence de VP4 de CVB4E2 ou de VP4 de CVB3
Figure 6 : Plasmide pMAL-c2
Figure 7 : Production d'IFNα lors de l'infection des PBMC par CVB4E2 préalablement incubé en présence de plasma de sujets sains et éventuellement de VP4 recombinante
   Légende de la Figure 7
   1. Milieu RPMI
   2. Plasma
   3. CVB4E2
   4.VP4 1µg/ml
   5. Plasma + VP4
   6. Plasma + CVB4E2
   7. Plasma + VP4 0.01µg/ml + CVB4E2
   8. Plasma + VP4 0.1µg/ml + CVB4E2
   9. Plasma + VP4 1µg/ml + CVB4E2
   10.Plasma + VP4 10µg/ml + CVB4E2
Figure 8 : Test ELISA comparatif entre VP4 et les peptides VP4-P1, VP4-P2, VP4-P3, VP4-P5, VP4-P6.
Figure 9 : Test ELISA comparatif entre VP4 et le peptide VP4-P2 (patient = moyenne de 40 sérums de patients diabétiques, control = moyenne de 40 sérums de patients non-diabétiques)
Figure 10 : Test ELISA comparatif entre VP4, VP4 recombinante et le peptide VP4-P2 (patient = moyenne de 40 sérums de patients diabétiques, control = moyenne de 40 sérums de patients non-diabétiques)
Figure 11 : Production d'IFNα lors de l'infection des PBMC par CVB4E2 préalablement incubé en présence de plasma de sujets sains et éventuellement de VP4
   Légende de la Figure 11 :
   1 : Milieu
   2 : P2(1µg/ml)
   3 : Plasma 1/100e
   4 : CVB4E2
   5 : CVB4E2 + Plasma 1/100e + Milieu
   6 : CVB4E2 + Plasma 1/100e + VP4(1µg/ml)
   7 : CVB4E2 + Plasma 1/100e + VP4-P2(1mg/ml)
   8 : CVB4E2 + Plasma 1/100e + VP4-P2(0,1mg/ml)
   9 : CVB4E2 + Plasma 1/100e + VP4-P2(0,01mg/ml)

### Exemple 1: Mise en évidence de la protéine virale de CVB3 ou CVB4 induisant des anticorps "facilitants" anti-CVB3 ou anti-CVB4 :

### a) Culture et purification des virus CVB3 et CVB4:

CVB3 (American Type Culture Collection, Manassas, USA) et CVB4 E2 diabétogène (fourni par Ji-Won Yoon, Julia McFarlane Diabetes Research Center, Calgary, Alberta, Canada) ont été cultivés dans les cellules Hep-2 (Biowhittaker, Verviers, Belgique) en milieu essentiel minimum de Eagle (Gibco BRL, Eragny, France) additionné de 10% de sérum de veau foetal inactivé par la chaleur (Gibco BRL) et 1% de L-Glutamine (Eurobio, France). Après une incubation de 24 heures à 37°C, 5% CO₂, la suspension cellulaire a été congelée et décongelée trois fois et centrifugée à 2000 x g pendant 10 min. Le virus issu du surnageant a été culotté par centrifugation à 500 000 x g pendant 3 heures à 4°C dans un rotor Beckman TLA-100.4. Le culot a été resuspendu dans 3 ml de Tris-HCl 0,01 M pH 7.2, contenant 0.5% (vol/vol) Nonidet P40, incubé à 4°C pendant 20 heures, homogénéisé, et centrifugé à 4000 x g pour ôter les débris insolubles. Puis 0,5 ml de la suspension virale clarifiée a été disposé en couches sur 0,5 ml de sucrose (30%, poids/vol) et 3 ml CsCl (40%, poids/vol). Après centrifugation à 348,000 x g à 4°C dans un rotor Beckman TLA-100.4 pendant 4 h, les fractions du gradient ont été récupérées et les titres viraux des fractions du gradient ont été déterminées par le test de la dose infectieuse de 50% de la culture tissulaire (TCID₅₀) sur une culture confluente de cellules Hep-2. Les fractions contenant les titres au pic d'infectiosité ont été groupées, dialysées et équilibrées avec du Tris-HCl 0,01 M pH 7.2 par centrifugation à 4000 x g sur membrane Macrosep™ (pall Filtron Corporation, Saint Germain en Laye, France) à exclusion de poids moléculaire (MWCO) de 300 K. Des aliquots ont été stockés congelés à -80°C. Des préparations contrôles ont été obtenues par le même protocole excepté que les cellules Hep-2 étaient infectées uniquement par le solvant du virus.

### b) Purification de la protéine VP4 naturelle et de l'antigène H des virus CVB3 et CVB4

La protéine VP4 et l'antigène H ont été dissociés des virus complets CVB3 et CVB4 comme décrit précédemment dans la littérature pour le poliovirus (Maizel et al., 1967). Brièvement, les particules virales CVB purifiées et concentrées (~ 1 mg) ont été incubées à 56°C pendant 5 min dans 0,5 ml de tampon salin (NaCl 0.1 M, citrate de sodium 0.005 M, pH 7.0). Ce traitement résulte en la dissociation du virus en ARN viral, VP4 et l'antigène H. Puis la protéine VP4 a été séparée du mélange par centrifugation à 4000 x g sur membrane Macrosep™ à MWCO de 100 Kd. L'antigène H et l'ARN ont été retenus par la membrane, et VP4 est passée au travers de la membrane. L'ARN a été dégradé par ajout de 0,05 mg de ribonucléase A bovine pancréatique (Roche Molecular Biochemicals, Mannheim, Allemagne) et incubation à 37°C pendant 10 min. L'antigène H et VP4 ont été dialysés et équilibrés avec du tampon phosphate salin (PBS), pH 7.2, par centrifugation à 4000 x g sur membranes Macrosep™ à MWCO de 100 Kd et 3 Kd respectivement. L'antigène H et VP4 ont été concentrés en utilisant un Savant Speed Vac Concentrator SVC100H (Global Medical Instrumentation, Minnesota, USA). La dissociation contrôle a été de même opérée avec le surnageant de cellules Hep-2 non-infectées purifié tel que décrit ci-dessus et a fourni des protéines contrôles pour ce qui suit. La concentration protéique a été calculée à partir de l'absorbance à 280 nm avec un coefficient d'extinction de 1 mg/ml.

### c) Inhibition par la protéine VP4 de la production d'IFNα induite par le couple CVB/plasma :

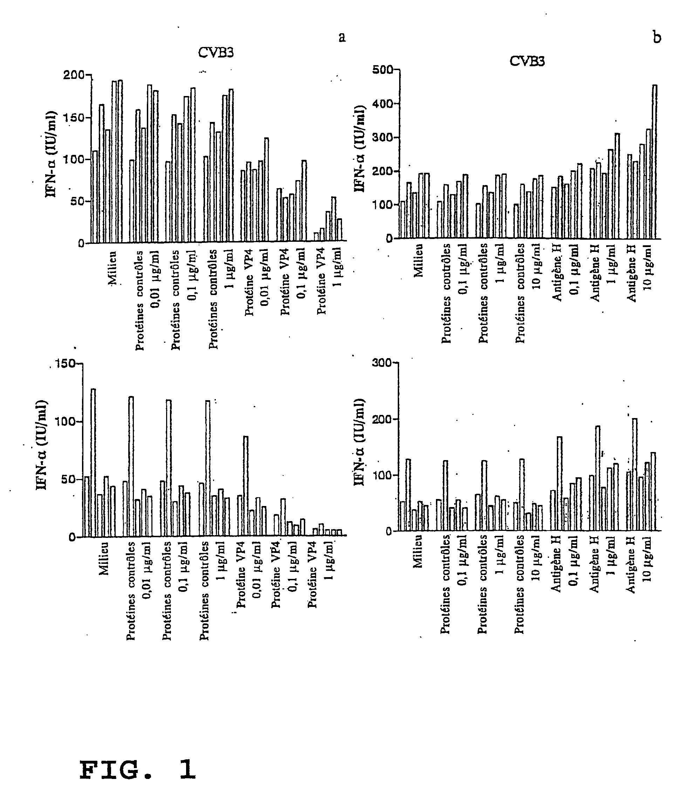

VP4, l'antigène H ou des protéines contrôles ont été préincubés pendant 1h à 37°C à différentes concentrations avec du plasma de 5 sujets sains dilués à une dilution optimale (1/10 ou 1/100). CVB3 ou CVB4 ont alors été ajoutés pendant 1h avant l'incubation avec des PBMC. On observe sur la Figure 1, une diminution dose-dépendante de la production d'IFNα induite par CVB3/plasma en présence de la protéine VP4_{CVB3} alors qu'en présence de l'antigène H on n'observe pas de diminution dose-dépendante de la production d'IFNα (Figure 1a et 1b). Des résultats similaires ont été obtenus avec la protéine VP4_{CVB4} dans le système CVB4/plasma (Figure 1c et 1d). Au contraire, les protéines contrôles isolées à partir de cellules Hep-2 non infectées n'ont eu aucun effet sur la production d'IFNα.

### Exemple 2: Diagnostic d'une infection à CVB3 ou CVB4 grâce à la détection des anticorps anti-VP4 - Corrélation entre la détection des anticorps anti-VP4 de CVB3 et CVB4 et le diabète de type 1

Pour détecter les anticorps anti-VP4 dans le plasma d'un donneur, des plaques de microtitration de 96 puits ont été incubées pendant une nuit à température ambiante avec la protéine VP4 dissociée à partir de CVB3 ou CVB4 à 5 µg/ml dans du PBS pH 7.4. De la même façon, des plaques de microtitration ont été incubées avec des protéines contrôles. Les puits ont ensuite été lavés trois fois avec une solution de lavage (PBS pH 7.4, 0,05% Tween 20), saturés pendant 1 h à 37°C avec du tampon de saturation (PBS, pH 7.4, 2,5% lait écrémé, 0,5% Tween 20), et lavés à nouveau 4 fois. Ensuite, 0,1 ml des échantillons, dilués dans du tampon de saturation à une dilution optimale (1/50), ont été ajoutés aux micropuits. Après 2 h d'incubation à température ambiante, les puits ont été lavés 4 fois et 0,1 ml du mélange d'anticorps IgA, G, M anti-humains marqués à la HRP ("horseradish peroxydase") dilué au 1/10000ème ont été ajoutés et incubés pendant 1 h. Après 4 cycles de lavage, 0,1 ml de la solution substrat (0,4 mg/ml o-phénylènediamine, 0,012% H₂O₂ dans du tampon phosphate citrate 0,05 M, pH 5.0) ont été ajoutés pendant 30 min. La réaction est arrêtée par l'addition de 25 µl d'acide sulfurique 2 N. Les mesures de l'absorbance ont été effectuées à 490 nm dans un lecteur de microplaques Dynex MRX® (Thermo Life Science, Cergy-Pontoise, France). La valeur limite du test immunologique a été déterminée en additionnant l'absorbance de l'échantillon sur les plaques contrôles à celle du blanc sur les plaques VP4. Les échantillons du test avec un indice (ratio échantillon/valeur limite) supérieur à 1,0 ont été considérés comme positifs pour la présence d'anticorps anti-VP4.

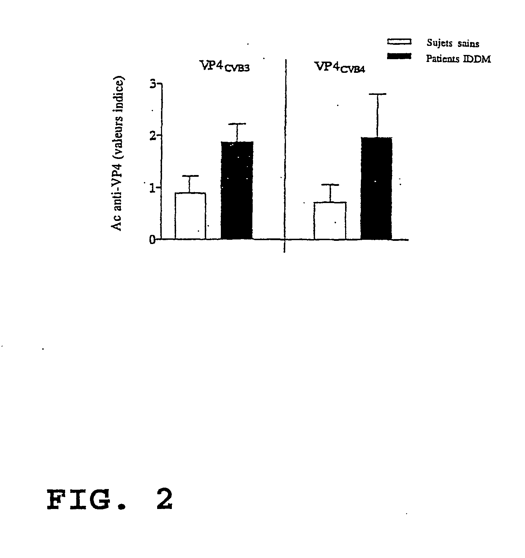

Ce test a été utilisé pour détecter et doser les anticorps anti-VP4 dans le plasma de sujets sains et de patients IDDM. 14 des 40 sujets sains (35%) et 25 des 40 patients IDDM (62,5%) étaient positifs pour les anticorps anti-VP4_{CVB3}. 6 des 40 sujets sains (15%) et 32 des 40 patients IDDM (80%) étaient positifs pour les anticorps anti-VP4_{CVB4}. La moyenne des indices obtenue dans le groupe des patients IDDM était significativement plus élevée que celle obtenue dans le groupe des sujets sains (Figure 2).

Le test développé dans cet exemple montre que la détection et le dosage des anticorps anti-VP4 peuvent être utilisés dans le diagnostic d'une pathologie associée à une infection aux coxsackievirus. En effet, les patients IDDM présentent une plus forte prévalence d'anticorps anti-VP4 et une plus grande quantité de ces anticorps que les sujets sains.

Certains patients présentent un taux d'anticorps anti-VP4 au-dessous du seuil de détection du test de l'exemple mais ceci peut s'expliquer par le fait que leur maladie est associée à d'autres virus que CVB3 ou CVB4 comme CVB2.

### Exemple 3 : Etude de la spécificité des anticorps "facilitants" au niveau du sérotype :

### A) Matériel et méthodes

### 1-Production d'IFNα par des PBMC en culture :

Les PBMC séparées du sang total hépariné sont distribuées dans des micropuits (plaque de 96 puits), à raison de 5 à 8.10⁵ cellules dans 100 µl de milieu RPMI supplémenté par puits. L'infection des PBMC se fait avec un volume final de 100 µl de la préparation dans chaque puits. Après une incubation de 48h à 37°C sous une atmosphère à 5% de CO₂ et 90% d'humidité, le surnageant est récolté et utilisé immédiatement pour le dosage d'IFNα, ou clarifié par 10 minutes de centrifugation à 180xg et gardé à -80°C jusqu'au dosage de l'IFNα produit.

La concentration d'IFNα produite est déterminée par une technique sensible et spécifique, la méthode DELFIA (Dissociation Enhanced Lanthanide FluoroImmunoAssay) (Rönnblom et al 1997).

### 2-Révélation de l'IFNα produit : méthode immunofluorométrique DELFIA

Le dosage de l'IFNα est réalisé selon le principe de DELFIA (Dissociation Enhanced Lanthanide FluoroImmunoAssay), basé sur une méthode d'immunofluorescence en phase retardée à l'aide d'anticorps liants l'IFNα dont un est marqué à l'Europium. L'utilisation d'une solution d'activation va libérer l'Europium conjugué à l'anticorps et émettre une fluorescence proportionnelle à la quantité d'IFNα produite et mesurée au fluoromètre (fluorometer LKB Wallac 1230 ARCUS^{®}, Turku, Finlande).

Les anticorps monoclonaux LT 273 (5,4 mg/ml) et LT 293 (4,8 mg/ml) anti-IFNα, fournis par le Dr Gunar Alm (Uppsala, Suède) sont fixés au fond des puits après une incubation de 12 heures à température ambiante. Ces plaques peuvent être conservées dans un tampon à 4°C ou utilisées immédiatement. Les échantillons standards d'IFNα humain sont préparés avec un anticorps monoclonal IgG1 « irrelevant » de souris pour établir la courbe de référence (10 mesures). Les autres échantillons à analyser sont déposés dans chaque puits (100 µl) avec le tampon de dilution et l'anticorps « irrelevant » et incubés 2 heures sous agitation douce à température ambiante. Les puits sont lavés 3 fois avec la solution de lavage. L'anticorps conjugué à l'Europium (200 µl) est déposé dans chaque puits en laissant incuber 1 heure à température ambiante sous agitation douce. La plaque est lavée 6 fois avec la solution de lavage. La solution d'activation (200 µl) ajoutée immédiatement après le lavage et laissée 20 à 30 minutes en incubation dans les puits provoque un clivage de l'Europium lié à l'anticorps fixé sur l'IFNα, émettant une fluorescence mesurée par le lecteur de fluorescence (LKB Wallac 1230 ARCUS^{®}). La concentration d'IFNα sera calculée à partir des valeurs de fluorescence mesurées grâce au logiciel Graphpad (San Diego, USA). Le seuil de détection d'IFNα est de 0,5 UI/ml.

### B)Résultats :

### 1- Evaluation de la production d'IFNα par les PBMC en présence de plasmas et pour chaque sérotype du coxsackie B :

Les plasmas de donneurs (dilution optimale au 1/10^{e} ou 1/100^{e}) sont pré-incubés 1 heure à 37°C avec les différents sérotypes du coxsackie virus B, CVB1 à CVB6 dilués au 1/10^{e}. Puis l'infection des cellules mononucléées du sang périphériques (PBMC) est effectuée. La révélation de la production d'IFNα obtenue est effectuée par méthode immunofluorométrique DELFIA après 48 heures d'infection des PBMC. Les mêmes plasmas sont utilisés à des dilutions identiques pour étudier la production d'IFNα par sérotype. La quantité de PBMC varie de 5.10⁵ à 7.10⁵/puits. Les plasmas de quatre donneurs sains différents ont été utilisés.

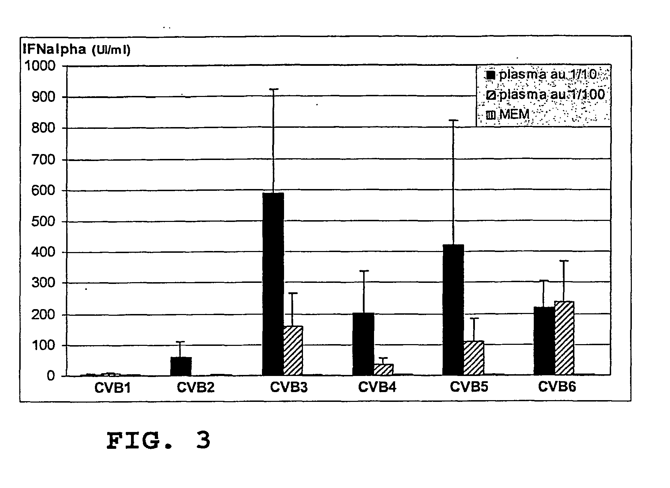

### Chaque sérotype de CVB entraîne une production d'interféron alpha en présence de plasma (voir Figure 3). Aucune production d'IFNα n'est obtenue en l'absence de plasma lors de ces expériences. La dilution du plasma au 1/10^{e} est à l'origine d'une production d'IFNα en moyenne plus importante que la dilution au 1/100^{e} (sauf pour CVB6). En présence de plasma le virus CVB1 reste par contre un faible inducteur d'IFNα.

### 2- Evaluation de la spécificité des anticorps facilitants par l'étude de la production d'IFNα lors de l'infection des PBMC par les différents sérotypes de CVB en présence de VP4-CVB4E2:

Chaque sérotype de CVB a été incubé une heure soit avec du plasma seul dilué au 1/10^{e}, soit avec du plasma dilué au 1/10^{e} préalablement incubé une heure avec la protéine VP4 recombinante (cf. Exemple 4) de CVB4E2 diluée au 1/10^{e}, soit avec du MEM.

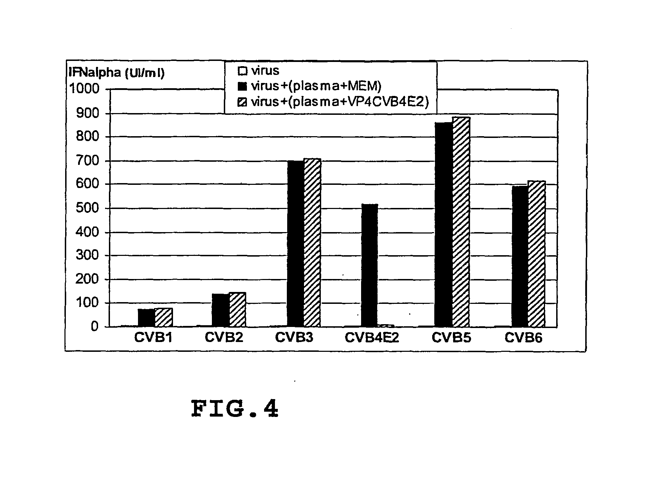

Les résultats montrent comme précédemment une production d'IFNα lors de l'infection des PBMC en présence de plasma. En pré-incubant le plasma avec la protéine VP4 de CVB4E2 cette production d'IFNα reste élevée et stable par rapport aux résultats précédents, sauf en présence de CVB4E2 où la production d'IFNα s'est effondrée. Les anticorps facilitants l'induction d'IFNα par CVB4E2 semblent donc spécifiquement dirigés contre la protéine VP4 de CVB4E2. (voir Figure 4)

### 3- Evaluation de la spécificité des anticorps facilitants par l'étude de la production d'IFNα lors de l'infection des PBMC par CVB4E2 ou CVB3 en présence de VP4 de CVB4E2 ou VP4 de CVB3 :

Le plasma d'un donneur sain (dilution optimale au 1/10^{e} ou 1/100^{e}) est pré-incubé 1 heure à 37°C avec la protéine recombinante VP4 de CVB4E2 (concentration optimale à 1 µg/ml). L'ensemble est à nouveau incubé une heure à 37°C en présence du virus CVB4E2 (dilutions aux 1/10^{e} et 1/100^{e} ; titre viral à 10¹³ TCID₅₀/ml) puis déposé sur les PBMC, (5.10⁵/puits) pour une incubation de 48 heures. La production d'IFNα par les PBMC est révélée par la méthode fluorométrique DELFIA après les 48 heures d'incubation.

La même expérience est effectuée avec le même lot de plasma (dilution optimale), la protéine naturelle VP4 de CVB3 (dilution 1/10^{e}) et le virus CVB3 (dilutions aux 1/10^{e} et 1/100^{e}; titre viral à 10¹³ TCID₅₀/ml). La détermination de la quantité d'IFNα produite par les PBMC est aussi faite à 48 heures par méthode DELFIA.

Des réactions croisées sont effectuées sur le même principe pour évaluer la spécificité des anticorps facilitant l'infection. Le mélange plasma et VP4 de CVB4E2 pré-incubée pendant 1 heure est ensuite incubée avec CVB3 avant d'être déposée sur les PBMC pendant 48 heures.

De la même façon, la préparation pré-incubée 1 heure de plasma et VP4 de CVB3 est incubée 1 heure avec CVB4E2 avant d'infecter les PBMC pendant 48 heures et de révéler la quantité d'IFNα produite.

Les PBMC infectées par les virus (CVB4E2 ou CVB3) en présence de plasma constituent les témoins positifs de cette expérimentation. Les témoins négatifs sont obtenus avec un puits de PBMC seules, un puits du virus CVB4E2 seul et un puits de CVB3 seul.

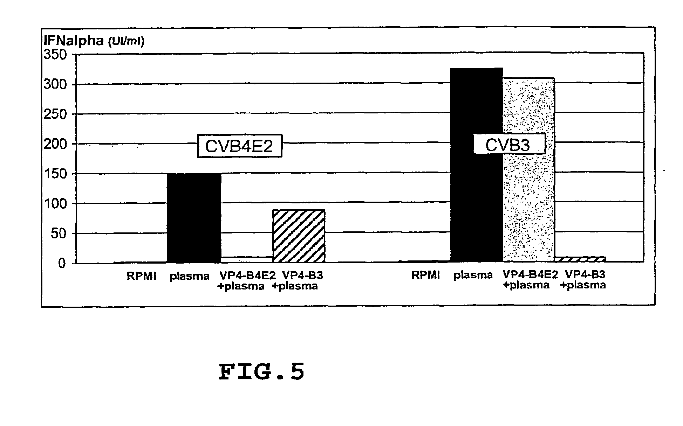

L'infection des PBMC par le virus CVB3 incubé une heure avec du plasma de donneur sain entraîne une production d'IFNα de 323 UI/ml (témoin positif). Lorsque le même plasma de donneur sain a été pré-incubé une heure avec la protéine VP4 de CVB4E2 avant de les mettre en présence du virus CVB3 pendant une heure on ne retrouve pas de différence de production d'IFNα (308 UI/ml). Par contre, lorsque le même plasma est pré-incubé initialement une heure avec la protéine VP4 spécifique de CVB3, avant d'être ajouté à CVB3, la production d'IFNα chute à 6,4 UI/ml, soit à 2% de sa valeur initiale (voir Figure 5).

L'infection des PBMC par le virus CVB4E2 incubé une heure avec du plasma de donneur sain entraîne une production d'IFNα de 148 UI/ml (témoin positif). Lorsque le même plasma de donneur sain est pré-incubé une heure avec la protéine VP4 de CVB3 avant de les mettre en présence du virus CVB4E2, on retrouve une production élevée d'IFNα par les PBMC (87 UI/ml). Par contre lorsque le même plasma est pré-incubé initialement une heure avec la protéine VP4 de CVB4E2 avant d'ajouter le virus CVB4E2, la production d'IFNα par les PBMC est faible à 8,5 UI/ml.

Les témoins virus seuls (CVB4E2 ou CVB3), protéines VP4 de CVB3 ou de CVB4E2 seules, MEM ou plasma seuls n'entraînent pas de production d'IFNα par les PBMC.

### Exemple 4 : Synthèse de la protéine VP4 de CVB4E2 recombinante :

### A) Production de VP4 recombinante

### 1-Bactéries utilisées.

Des bactéries *Escherichia coli* compétentes JM 109 (Promega, Madison, Etats-Unis) sont utilisées.

### 2-Plasmide utilisé.

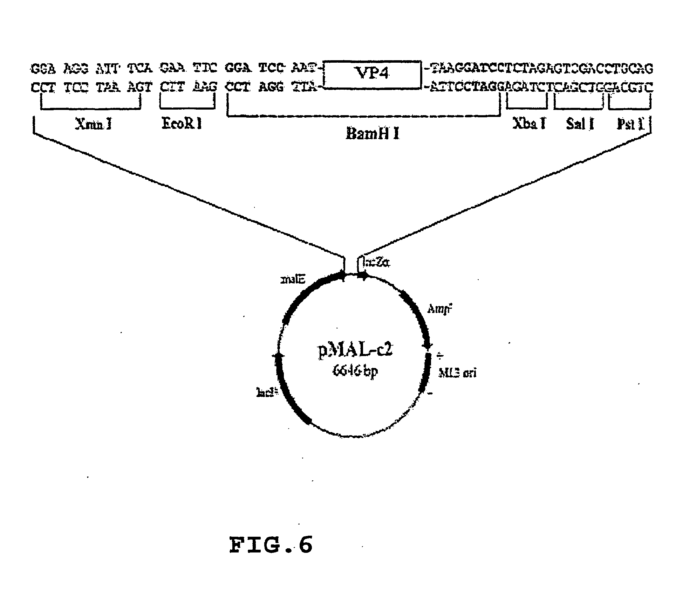

Le plasmide utilisé pour transformer les bactéries compétentes est pMAL-c2 (Valera-Calvino et al, 2000) fournit par le docteur Ruben Valera Calvino, plasmide codant une beta-lactamase, d'où la sélection des bactéries ayant reçu le plasmide sur un milieu contenant de l'ampicilline, et codant la protéine MBP, à laquelle a été fusionnée la protéine VP4, sous le contrôle d'un promoteur inductible à l'IPTG (Promega, Madison,Etats-Unis).(voir Figure 6)

### 3- Milieux utilisés.

### α. Milieu SOC.

Ce milieu comprend, pour un litre, 20 g de Bacto™ tryptone (DIFCO-BECTON DICKINSON, Etats-Unis), 5 g d'extrait de levure (DIFCO, Detroit, Etats-Unis),10 ml de NaCl 1 M, 2,5 ml de KCl 1 M, 10ml de MgCl₂ 1M/MgSO₄ 1M et 10 ml de glucose 2M, le pH est ajusté à 7.

### β. Milieu LB.

Ce milieu comprend, pour un litre, 10 g de tryptone (DIFCO-BECTON DICKINSON, Etats-Unis), 5 g d'extrait de levure (DIFCO, Detroit, Etats-Unis), 5 g de NaCl , son p.H est 7,2. Pour les boites de milieu LB, ce milieu comporte également 15 g d'agar contenant de l'ampicilline (Invitrogen, Cergy Pontoise, France). Le milieu LB utilisé pour la production de protéines recombinantes est enrichi, par ajout de 2 g de glucose par litre et contient 100 µg d'ampicilline par litre

### 4-Tampons utilisés

### α-Tampon de colonne.

Cette solution comprend, pour un litre : 20 ml tris-HCl (Q.BIOgene, Etats-Unis) 1M pH 7,4, 11,7 g de NaCl, 2 ml d'EDTA 0,5 M (Sigma-Aldrich, Etats-Unis). Pour l'élution de la protéine MBP-VP4, 3,6 g de maltose (Sigma-Aldrich, Etas-Unis)(10 mM) sont ajoutés dans le tampon de colonne alors appelé tampon d'élution.

### β-Tampon de digestion.

Cette solution comprend, pour un litre : 3,2 g de tris-HCl (Q.BIOgene) (0,2 M), 5,84 g de NaCl (100mM) et 0,22 g de CaCl₂, le pH est ajusté à 8.

### 5-Transformation des bactéries.

Un aliquot de bactéries compétentes est décongelé dans la glace, puis, 1 à 2 µl du plasmide sont ajoutés, et incubés avec les bactéries 30 minutes dans la glace. Les bactéries subissent alors un choc thermique : elles sont mises 30 s à 42°C, avant de retourner 1 à 2 minutes dans la glace. 250µl de milieu SOC porté à la température de la pièce est ajouté, le tube est incubé 1 heure à 37°C sous agitation. 20 à 100 µl de la suspension de bactéries sont étalés sur une boite de milieu contenant de l'ampicilline, pour sélectionner les bactéries transformées, la boite est incubée une nuit à 37°C.

Des volumes différents de la suspension de bactéries sont étalés sur différents boites, afin d'obtenir au moins une boite où les colonies sont isolées. Les boites avec les colonies sont stockées à 4°C.

### 6-Production de la protéine recombinante MBP-VP4.

10 ml de milieu LB riche sont inoculés avec une colonie de bactéries transformées dans un tube Falcon® 15 ml, qui est incubé une nuit à 37°C. Le lendemain, dans une flasque de 2,5 litre, 1 litre de milieu LB riche est inoculé avec ces 10 ml, puis incubé à 37°C sous agitation, jusqu'à ce que sa D.O (densité optique) atteigne une valeur comprise entre 0,5 et 0,6. Alors, la production de MBP-VP4 est induite par ajout de 3 ml d'IPTG (isopropylthiogalactoside) dans la flasque, qui est incubée au moins 3 heures à 37°C sous agitation.

### 7-Récupération de la protéine MBP-VP4 et obtention de VP4.

La culture de bactéries est centrifugée 20 minutes à 4000xg à 4°C. Le surnageant est ensuite éliminé et le culot de bactéries est repris dans 50 ml de tampon de colonne et congelé la nuit à -20°C, avant d'être décongelé dans l'eau froide et placé dans la glace le lendemain matin. Les bactéries sont lysées par 8 sonications successives de 15 secondes. Le lysat bactérien est clarifié par une centrifugation 30 minutes à 9000xg, à 4°C, le surnageant étant récupéré. En parallèle, 5 ml de résine d'amylose (NEW ENGLAND BioLabs, Etats-Unis) sont placés dans la colonne à chromatographie, puis lavés avec 40 ml de tampon de colonne. L'extrait cellulaire contenant la protéine de fusion MBP-VP4 est injecté dans la colonne avec un débit de 25,6 ml par heure. La résine est lavée par 60 ml de tampon de colonne. La protéine MBP -VP4 est éluée avec le tampon d'élution, les fractions sont récupérées à raison de 1 ml par fraction, Les fractions ayant une D.O. à 280nm maximale sont regroupées. La protéine est déssalée contre du PBS, par dialyse et concentrée sur une membrane filtre Macrosep™ à 10 Kd (PALL, Life Sciences, Etats-Unis) par centrifugation à 5000xg, à 4°C, au moins 30 minutes.

La protéine MBP-VP4 obtenue est clivée avec le facteur Xa (NEW ENGLAND BioLabs, Etats-Unis), dont la concentration optimale est 2% de celle de la protéine de fusion (déterminée par spectrométrie U.V, avec l'absorbance à 280 nm), dans du tampon d'élution ou du tampon de digestion.

La protéine MBP, la MBP-VP4 non clivée et le facteur Xa sont éliminés par centrifugation sur une membrane filtre Macrosep™ à 10 Kd perméable seulement à la protéine VP4. Le filtrat est récupéré et concentré par centrifugation sur une membrane filtre Macrosep™ à 3 Kd (PALL, Life Sciences, Etats-Unis). La protéine VP4 concentrée est stockée à -80°C.

### B)Compétition entre la protéine VP4 et le virus CVB4E2 en présence de plasma :

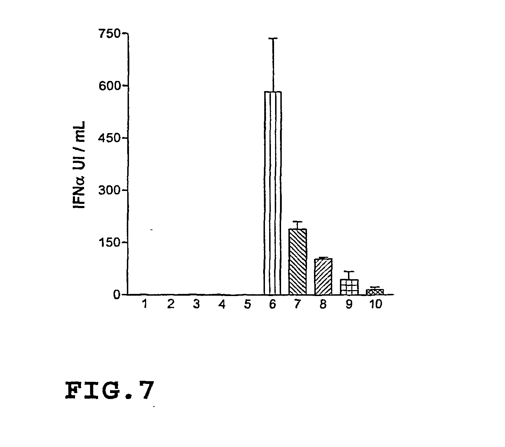

On mesure la production d'IFNα induite dans des cultures de PBMC par CVB4E2 préalablement incubé en présence de plasma de sujets sains. Ce niveau de production d'IFNα est mesuré par la méthode DELFIA dans les surnageants de culture récupérés au bout de 48 heures. Chaque condition de culture nécessite 2.10⁵ cellules par puits, et une m.o.i. de 1 pour CVB4E2. Les échantillons de plasma sont dilués au dixième dans du milieu RPMI et sont incubés 1 heure avec CVB4E2 à 37°C. la protéine VP4 recombinante est mise en présence de plasma pendant une heure à 37°C avant l'incubation avec CVB4E2. Les résultats présentent les moyennes et écarts types de trois expériences indépendantes réalisées avec trois donneurs différents.(voir Figure 7)

La protéine VP4 recombinante, incubée avec le plasma à des taux variables (10, 1, 0.1, 0.01µg/ml) permet de constater un effet dose-réponse de la protéine VP4 recombinante sur l'effet procuré sur l'incubation avec CVB4E2 des PBMC par la préincubation du plasma avec le virus. On obtient ainsi avec la protéine à un taux de 0.01µg une production d'IFNα de 189.5 +/- 21.6 UI/mL, avec un taux de 0.1µg/ml 104.7 +/- 4.5 UI/mL, avec un taux de 1µg/ml 44.1 +/- 24.3 UI/mL d'IFNα et avec un taux de 10µg/ml 15.3 +/- 7.9 UI/mL. Ainsi plus la dose de VP4 recombinante incubée avec le plasma est importante, plus les valeurs d'IFNα sont faibles (voir Figure 7).

### Exemple 5 : Recherche d'un fragment peptidique de la protéine VP4 de CVB4E2 mimant l'activité biologique de la protéine VP4 de CVB4E2 pleine longueur :

### A)Matériels et méthodes (test ELISA)

### 1- Sérums et plasmas testés :

40 plasmas de patients non diabétiques et 40 plasmas ou sérums de patients diabétiques ont été testés.

### 2- Peptides VP4 utilisés lors des tests ELISA :

Les 5 peptides VP4 utilisés ont été synthétisés par la société EPYTOPE (Nimes) sur base de la séquence de la protéine VP4 du virus CVB4-E2 répertoriée dans la banque de donnée NCBI (N° d'accession de la séquence : Q86887). Leur position sur la séquence de la protéine VP4 du virus CVB4-E2 et leur séquence sont présentées dans le Tableau 1 ci-dessous. Ils sont chevauchants et couvrent la totalité de la séquence de la VP4.

**Tableau 1 :**

| Nom | SEQ ID N° | Séquence | Position |
|---|---|---|---|
| VP4-P1 | 1 | NH2-MGAQVSTQKTGAHETSLSAS-CONH2 | aa1-20 |
| VP4-P2 | 2 | NH2-GAHETSLSASGNSIIHYTNI-CONH2 | aa11-30 |
| VP4-P3 | 3 | NH2-GNSIIHYTNINYYKDAASNS-CONH2 | aa21-40 |
| VP4-P5 | 4 | NH2-NYYKDAASNSANRQDFTQDPSKFTEPVKDV-CONH2 | aa31-60 |
| VP4-P6 | 5 | NH2-SKFTEPVKDVMIKSLPALN-CONH2 | aa51-69 |

### 3- Composition des tampons utilisés :

### - Tampon PBS : (pour un litre)

* NaCl 8.0 g
* KCl 0.2 g
* KH2PO4 0.2 g
* . Na2HPO4/2H2O 1.44 g
Ajuster le pH à 7.4

### - Solution de lavage :

PBS pH=7.4 contenant 0.05% de tween 20.

### - Tampon de saturation et de dilution :

Milk diluent/blocking solution concentrée (KPL) diluée au 1/20^{ème} dans l'eau.

### - "Post-coating buffer :

Tampon phosphate de sodium 50 mM pH=4.5 contenant
* D-Sorbitol 6%
* NaCl 0.9%
* BSA 0,1%
* CAC12/2 H2O 0.1 mM
* EDTA 4 µM
* NaN3 0.005%
Ajuster le pH à 4.8

### - Solution d'anticorps secondaire :

Anticorps anti-IgG.A.M (H+L) humain couplé à la péroxidase (Bio-Rad) dilué à 1/10 000^{ème} dans le tampon de dilution

### - Tampon de révélation :

Dissoudre une capsule Phosphate citrate buffer (Sigma) dans 100 ml d'eau distillée (Cf = 0.05 M, pH 5.0). Prélever 25 ml de ce tampon et les placer dans un flacon de 50 ml entouré d'aluminium. Prendre une pastille d'ODP à 10 mg (Sigma) et la dissoudre dans ces 25 ml (Cf = 0.4 mg/ml). Mélanger à l'aide d'un vortex. Juste avant utilisation ajouter 10µl d'H₂O₂ à 30%.

### 4- Préparation des plaques ELISA :

La protéine VP4 ou les 5 peptides VP4, dilués à 5µg/ml en tampon PBS pH=7.4, sont déposés dans les puits d'une microplaque de 96 puits (FluoroNunc, MaxiSorp surface, NUNC, Danemark) à raison de 100µl/puits. Les plaques sont ensuite incubées 1h à 37°C sous atmosphère humide, puis laissées 24h à température ambiante. Après 3 lavages avec le tampon PBS, les puits sont saturés avec 300µl de tampon de saturation, pendant 1h à 37°C sous atmosphère humide, avant d'être lavés 3 fois avec le tampon de lavage. 250µl de post-coating buffer sont ensuite déposés dans chacun des puits et les plaques sont incubées pendant 24h à température ambiante puis conservées à 4°C jusqu'à leur utilisation.

### 5- Dosage ELISA :

Les puits coatés avec la protéine VP4 ou les différents peptides VP4 sont lavés 3 fois avec le tampon de lavage afin d'éliminer la solution de post-coating. Puis 100µl des plasmas ou sérums dilués au 1/50^{ème} dans le tampon de dilution sont déposés dans les différents puits et les plaques sont incubées 1h à 37°C sous atmosphère humide. Un puits témoin négatif contenant 100µl de tampon de dilution est réalisé en parallèle. Les puits sont ensuite lavés 3 fois avec la solution de lavage et 100µl de solution d'anticorps secondaire sont ajoutés dans chacun des puits. Après incubation 1h à 37°C sous atmosphère humide, les puits sont lavées 6 fois avec la solution de lavage et 100µl de solution de substrat sont ajoutés dans chacun des puits. Les plaques sont ensuite incubées 30 minutes à température ambiante et à l'obscurité. La réaction colorée est stoppée en ajoutant 25µl d'H₂SO₄ 2N (1M) par puits et les plaques sont lues à l'aide d'un lecteur de plaques à la longueur d'onde de 490nm.

### B)Résultats par ELISA de la comparaison entre la protéine VP4 et les peptides VP4-P1, VP4-P2, VP4-P3, VP4-P5, VP4-P6 :

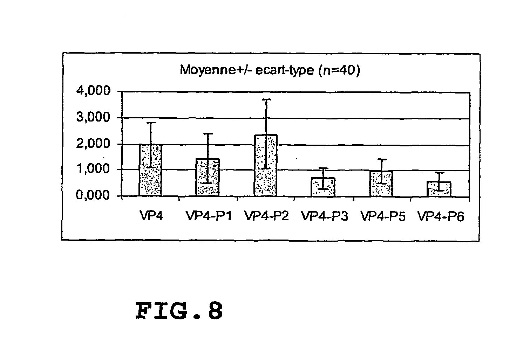

Les DO obtenues pour chacun des sérums avec la protéine VP4 et les différents peptides VP4 ont été divisées par la DO obtenue avec le témoin négatif. Les tableaux ci-dessous présentent la moyenne +/- écart-type des rapports de DO obtenus avec les deux séries de sérums testés (Patient = patients diabétiques, Control = non diabétiques). (voir Figures 8 à 10)

**Tableau 2 : Résultats du Test ELISA VP4/peptides VP4 (voir Figure 8)**

| | VP4 | VP4-P1 | VP4-P2 | VP4-P3 | VP4-P5 | VP4-P6 |
|---|---|---|---|---|---|---|
| Moyenne | 1,964 | 1,450 | 2,373 | 0,712 | 0,968 | 0,600 |
| Ecart-type | 0,86466771 | 0,951 | 1,313 | 0,399 | 0,456 | 0,338 |

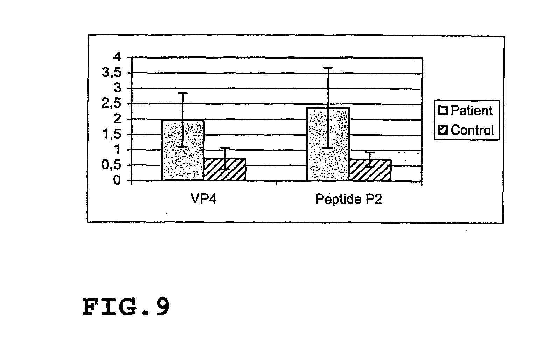

**Tableau 3 : Résultats du Test ELISA VP4/peptide VP4-P2 (voir Figure 9)**

| | | | | |
|---|---|---|---|---|
| | Moyenne | (n=40) | écart-type | |
| | VP4 | Peptide P2 | VP4 | Peptide P2 |
| Patient | 1,964 | 2,376 | 0,86466771 | 1,30832433 |
| Control | 0,711 | 0,691 | 0,348 | 0,23973485 |

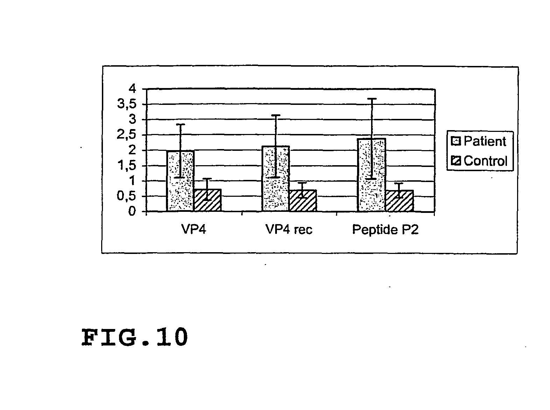

**Tableau 4 : Résultats du Test ELISA VP4/VP4 recombinante peptide P2 (voir Figure 10)**

| | Moyenne (n=40) | | | écart-type | | |
|---|---|---|---|---|---|---|
| Série | VP4 | VP4 rec | Peptide P2 | VP4 | VP4 rec | Peptide P2 |
| Patient | 1,964 | 2,119 | 2,376 | 0,865 | 1,009 | 1,308 |
| Control | 0,711 | 0,695 | 0,691 | 0,348 | 0,249 | 0,240 |

Les Figures 8 à 10 représentent schématiquement les résultats des tests ELISA.

Les peptides VP4-P1 (SEQ ID N°1) et VP4-P2 (SEQ ID N°2) montrent une DO comparable à la protéine VP4 dans le test ELISA avec 40 sérums de patients. (voir Figure 8)

En particulier, le peptide VP4-P2 (SEQ ID N°2) a le même comportement que VP4 dans le test ELISA comparant la moyenne des DO de 40 sérums de patients et de 40 sérums de non-diabétiques. (voir Figures 9 et 10) Le peptide VP4-P2 (SEQ ID N°2), comme VP4, reconnaît donc plus d'anticorps chez les diabétiques que chez les non-diabétiques.

### C)Résultats du test biologique (production d'IFNα par des PBMC) de la comparaison entre la protéine VP4 et le peptide VP4-P2 :

Le matériel et méthodes pour la production d'IFNα par des PBMC en culture et pour la révélation de l'IFNα produit par la méthode DELFIA est le même que celui de l'Exemple 3.

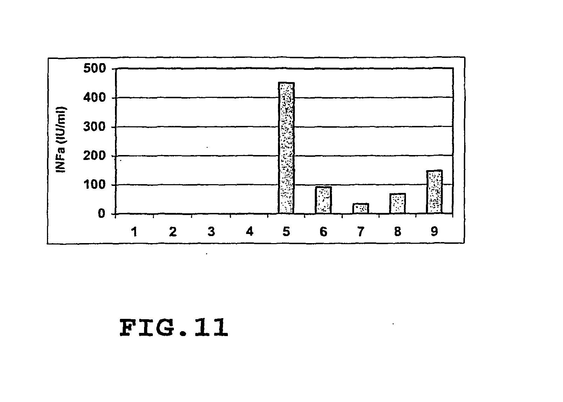

Le peptide VP4-P2 (SEQ ID N°2) montre une diminution dose-dépendante de la production d'IFNα lors de l'infection des PBMC en présence de plasma. (voir Figure 11, pistes 7 à 9)

Le peptide VP4-P2 mime donc la protéine VP4 dans ce test biologique.

Ces résultats, corroborés aux tests ELISA (point B)), suggèrent que le peptide VP4-P2 (SEQ ID N°2) porte un ou des épitopes reconnus par les anticorps "facilitants" anti-VP4.

## Revendications

1. Test de diagnostic in vitro d'entérovirus basé sur la révélation d'une réaction immunologique de type reconnaissance antigène-anticorps **caractérisé en ce que** :
- l'antigène mis en oeuvre ou des épitopes de celui-ci, sont ceux qui n'induisent pas d'anticorps antiviraux neutralisants mais qui induisent des anticorps "facilitants" qui augmentent l'infection virale, et
- ledit antigène induisant des anticorps antiviraux « facilitants » est la protéine virale interne structurale VP4, ou un fragment de celle-ci.

2. Test de diagnostic selon la revendication 1 **caractérisé en ce que** la réaction immunologique de type reconnaissance antigène-anticorps est révélée soit par un anticorps anti-espèce marqué quand le test selon l'invention consiste en la détection des anticorps "facilitants" par les antigènes correspondants fixés sur un support, soit par un anticorps anti-viral marqué ou par un anticorps anti-viral puis un anticorps secondaire anti-espèce marqué quand le test selon l'invention consiste en la détection des antigènes par les anticorps "facilitants" correspondants fixés sur un support.

3. Test de diagnostic selon la revendication 2 **caractérisé en ce que** l'anticorps marqué porte un marqueur de type enzyme, radioisotope, composé fluorescent, composé chimioluminescent, composé bioluminescent ou chélate de métaux.

4. Test de diagnostic selon la revendication 1 ou 2, **caractérisé en ce que** la protéine virale interne ou un fragment de celle-ci est obtenu soit par purification à partir du virus, soit est une protéine recombinante présentant les mêmes propriétés immunogènes, soit est synthétisée chimiquement et présente les mêmes propriétés immunogènes.

5. Test de diagnostic selon la revendication 4 **caractérisé en ce que** le fragment de la protéine structurale VP4 est choisi parmi les peptides de séquence SEQ ID N°1, SEQ ID N°2, SEQ ID N°3, SEQ ID N°4, SEQ ID N°5.

6. Test de diagnostic selon la revendication 4 **caractérisé en ce que** le fragment de la protéine structurale VP4 est le peptide de séquence SEQ ID N°2.

7. Test de diagnostic selon l'une quelconque des revendications 1 à 6 **caractérisé en ce que** les entérovirus diagnostiqués sont les coxsackievirus de type A ou B

8. Test de diagnostic selon l'une quelconque des revendications 1 à 7 **caractérisé en ce que** sa mise en oeuvre comprend la succession des étapes suivantes:
a- immobilisation des anticorps "facilitants" ou de la protéine virale induisant des anticorps "facilitants" ou d'un fragment de celle-ci sur un support
b- immobilisation d'anticorps contrôles, de protéines contrôles ou d'un fragment de celles-ci sur un support
c- lavages avec une solution saline tamponnée additionnée ou non d'un détergent à faible concentration
d- saturation de la surface de support non recouverte par une solution tamponnée de protéines irrelevantes
e- lavages avec une solution saline tamponnée additionnée ou non d'un détergent à faible concentration
f- application des échantillons à étudier à différentes dilutions dans du tampon de saturation
g- lavages avec une solution saline tamponnée additionnée ou non d'un détergent à faible concentration
h- amplification de la réponse par application d'anticorps marqués
i- lavages avec une solution saline tamponnée additionnée ou non d'un détergent à faible concentration
j- lecture de l'intensité du marquage

9. Test de diagnostic selon l'une quelconque des revendications 1 à 8 **caractérisé en ce que** les antigènes ou les anticorps "facilitants" correspondants sont fixés sur un support de type plaque à puits multiples pour microtitration.

10. Test de diagnostic selon l'une quelconque des revendications 1 à 8 **caractérisé en ce que** les antigènes ou les anticorps "facilitants" correspondants sont fixés sur un support de type puce.

11. Coffret ou kit pour la mise en oeuvre d'un test de diagnostic in vitro d'entérovirus selon l'une quelconque des revendications 1 à 10 qui comprend :
- la protéine virale interne structurale VP4 ou un fragment de celle-ci ou les anticorps "facilitants" correspondants,
- les réactifs pour la constitution du milieu propice à la réalisation de la réaction antigène-anticorps,
- les réactifs permettant la détection du complexe formé.

12. Utilisation du test de diagnostic selon l'une quelconque des revendications 1 à 10 pour prédire le déclenchement d'une pathologie chronique humaine ou animale associée à une infection par un entérovirus.

13. Utilisation du test de diagnostic selon l'une quelconque des revendications 1 à 10 pour prédire le déclenchement d'un diabète de type 1 chez un patient prédiabétique.

14. Utilisation du test de diagnostic selon l'une quelconque des revendications 1 à 10 pour définir le stade d'une pathologie chronique humaine ou animale associée à une infection par un entérovirus.

15. Utilisation du test de diagnostic selon l'une quelconque des revendications 1 à 10 pour définir le stade d'un diabète de type 1 associé à une infection par CVB.

16. Utilisation du test de diagnostic selon l'une quelconque des revendications 1 à 10 pour déterminer le sérotype de l'entérovirus responsable d'une infection aigüe humaine ou animale.

17. Utilisation du test de diagnostic selon l'une quelconque des revendications 1 à 10 pour déterminer la répartition par sérotype des infections entérovirales dans une population donnée humaine ou animale.

18. Utilisation du test de diagnostic selon l'une quelconque des revendications 1 à 10 pour déterminer *in vitro* la cible virale d'un agent antiviral.

## Claims

1. An in vitro diagnostic test for enterovirus based on revealing an immunoreaction of antigen-antibody recognition type, **characterized in that**:
- the antigen used, or epitopes thereof, are those which do not induce neutralizing antiviral antibodies but which induce "facilitating" antibodies which increase the viral infection, and
- said antigen inducing "facilitating" antiviral antibodies is the internal structural viral protein VP4, or a fragment thereof.

2. A diagnostic test according to claim 1, **characterized in that** the immunoreaction of antigen-antibody recognition type is revealed either with a labelled anti-species antibody when the test according to the invention consists in detecting the "facilitating" antibodies by means of the corresponding antigens attached to a support, or with a labelled antiviral antibody or with an antiviral antibody and then a labelled anti-species secondary antibody when the test according to the invention consists in detecting the antigens by means of the corresponding "facilitating" antibodies attached to a support.

3. A diagnostic test according to claim 2, **characterized in that** the labelled antibody carries a label of enzyme, radioisotope, fluorescent compound, chemiluminescent compound, bioluminescent compound or metal chelate type.

4. A diagnostic test according to claim 1 or 2, **characterized in that** the internal viral protein or a fragment thereof is either obtained by purification from the virus, or is a recombinant protein having the same immunogenic properties, or is synthesized chemically and has the same immunogenic properties.

5. A diagnostic test according to claim 4, **characterized in that** the fragment of the structural protein VP4 is chosen from the peptides of sequence SEQ ID No. 1, SEQ ID No. 2, SEQ ID No. 3, SEQ ID No. 4 or SEQ ID No. 5.

6. A diagnostic test according to claim 4, **characterized in that** the fragment of the structural protein VP4 is the peptide of sequence SEQ ID No. 2.

7. A diagnostic test according to any one of claims 1 to 6, **characterized in that** the enteroviruses diagnosed are coxsackieviruses type A or B.

8. A diagnostic test according to any one of claims 1 to 7, **characterized in that** the implementation thereof comprises the succession of the following steps:
a- immobilizing the "facilitating" antibodies or the viral protein inducing "facilitating" antibodies or a fragment of said protein on a support;
b- immobilizing control antibodies or control proteins or a fragment of said proteins on a support;
c- washing with a buffered saline solution to which a detergent may or may not have been added at a low concentration;
d- saturating the support surface not coated, with a buffered solution of irrelevant proteins;
e- washing with a buffered saline solution to which a detergent may or may not have been added at a low concentration;
f- applying the samples to be studied at various dilutions in saturation buffer;
g- washing with a buffered saline solution to which a detergent may or may not have been added at a low concentration;
h- amplifying the response by applying labelled antibodies;
i- washing with a buffered saline solution to which a detergent may or may not have been added at a low concentration;
j- reading the intensity of the labelling.

9. A diagnostic test according to any one of claims 1 to 8, **characterized in that** the antigens or the corresponding "facilitating" antibodies are attached to a support of multiwell microtitration plate type.

10. A diagnostic test according to any one of claims 1 to 8, **characterized in that** the antigens or the corresponding "facilitating" antibodies are attached to a support of chip type.

11. A pack or kit for carrying out an in vitro diagnostic test for enterovirus according to any one of claims 1 to 10, which comprises:
- the internal structural viral protein VP4 or a fragment thereof or the corresponding "facilitating" antibodies,
- the reagents for constituting the medium suitable for carrying out the antigen-antibody reaction,
- the reagents for detecting the complex formed.

12. The use of the diagnostic test according to any one of claims 1 to 10, for predicting the triggering of a chronic human or animal pathology associated with an enterovirus infection.

13. The use of the diagnostic test according to any one of claims 1 to 10, for predicting the triggering of type 1 diabetes in a prediabetic patient.

14. The use of the diagnostic test according to any one of claims 1 to 10, for defining the stage of a chronic human or animal pathology associated with an enterovirus infection.

15. The use of the diagnostic test according to any one of claims 1 to 10, for defining the stage of a type 1 diabetes associated with a CVB infection.

16. The use of the diagnostic test according to any one of claims 1 to 10, for determining the serotype of the enterovirus responsible for an acute human or animal infection.

17. The use of the diagnostic test according to any one of claims 1 to 10, for determining the distribution by serotype of enteroviral infections in a given human or animal population.

18. The use of the diagnostic test according to any one of claims 1 to 10, for determining, *in vitro*, the viral target of an antiviral agent.

## Patentansprüche

1. Diagnostischer In-vitro-Test auf Enteroviren, der auf dem Nachweis einer immunologischen Reaktion des Typs Antigen-Antikörper-Erkennung beruht, **dadurch gekennzeichnet, dass**:
- das eingesetzte Antigen oder Epitope desselben die sind, die keine neutralisierenden antiviralen Antikörper induzieren, sondern "fördernde" Antikörper induzieren, die die Virusinfektion verstärken, und
- dieses "fördernde" antivirale Antikörper induzierende Antigen das strukturelle interne Virusprotein VP4 oder ein Fragment desselben ist.

2. Diagnostischer Test gemäß Anspruch 1, **dadurch gekennzeichnet, dass** die immunologische Reaktion des Typs Antigen-Antikörper-Erkennung entweder durch einen markierten Anti-Spezies-Antikörper, wenn der erfindungsgemäße Test aus der Erfassung der "fördernden" Antikörper durch die entsprechenden, auf einem Träger fixierten Antigene besteht, oder durch einen markierten antiviralen Antikörper oder durch einen antiviralen Antikörper und dann einen markierten sekundären Anti-Spezfes-Antikörper nachgewiesen wird, wenn der erfindungsgemäße Test aus der Erfassung von Antigenen durch die entsprechenden, auf einem Träger fixierten "fördernden" Antikörper besteht.

3. Diagnostischer Test gemäß Anspruch 2, **dadurch gekennzeichnet, dass** der markierte Antikörper eine Markierung vom Typ eines Enzym, Radioisotops, fluoreszierenden Verbindung, chemolumineszierenden Verbindung, biolumineszierenden Verbindung oder Metallchelats trägt.

4. Diagnostischer Test gemäß Anspruch 1 oder 2, **dadurch gekennzeichnet, dass** das interne Virusprotein oder ein Fragment desselben entweder durch Reinigung aus dem Virus erhalten wird oder ein rekombinantes Protein ist, das dieselben immunogenen Eigenschaften aufweist oder chemisch synthetisiert wird und dieselben immunogenen Eigenschaften autweist.

5. Diagnostischer Test gemäß Anspruch 4, **dadurch gekennzeichnet, dass** das Fragment des Strukturproteins VP4 aus den Peptiden der Sequenz SEQ ID Nr. 1, SEQ ID Nr. 2, SEQ ID Nr. 3, SEQ ID Nr. 4 und SEQ ID Nr. 5 ausgewählt ist.

6. Diagnostischer Test gemäß Anspruch 4, **dadurch gekennzeichnet, dass** das Fragment des Strukturproteins VP4 das Peptid der SEQ ID Nr. 2 ist.

7. Diagnostischer Test gemäß einem der Ansprüche 1 bis 6, **dadurch gekennzeichnet, dass** die diagnostizierten Enteroviren die Coxsackieviren des Typs A oder B sind.

8. Diagnostischer Test gemäß einem der Ansprüche 1 bis 7, **dadurch gekennzeichnet, dass** sein Einsatz die Abfolge der folgenden Schritte umfasst:
a - Immobilisierung der "fördernden" Antikörper oder des "fördernde" Antikörper induzierenden Virusproteins oder eines Fragments desselben auf einem Träger,
b - Immobilisierung von Kontrollantikörpern, Kontrollproteinen oder eines Fragments derselben auf einem Träger,
c - Waschen mit einer gepufferten Kochsalzlösung, die mit einem Waschmittel in geringer Konzentration ergänzt oder nicht ergänzt ist,
d - Sättigen der nicht belegten Trägeroberfläche mit einer gepufferten Lösung irrelevanter Proteine,
e - Waschen mit einer gepufferten Kochsalzlösung, die mit einem Waschmittel in geringer Konzentration ergänzt oder nicht ergänzt ist,
f- Aufbringen zu untersuchender Proben in verschiedenen Verdünnungen in Sättigungspuffer,
g - Waschen mit einer gepufferten Kochsalzlösung, die mit einem Waschmittel in geringer Konzentration ergänzt oder nicht ergänzt ist,
h - Verstärken der Antwort durch Anwendung markierter Antikörper,
i - Waschen mit einer gepufferten Kochsalzlösung, die mit einem Waschmittel in geringer Konzentration ergänzt oder nicht ergänzt ist, j - Ablesen der Markierungsintensität.

9. Diagnostischer Test gemäß einem der Ansprüche 1 bis 8, **dadurch gekennzeichnet, dass** die Antigene oder die entsprechenden "fördernden" Antikörper auf einem Träger des Multinäpfchenplattentyps zur Mikrotitration fixiert sind.

10. Diagnostischer Test gemäß einem der Ansprüche 1 bis 8, **dadurch gekennzeichnet, dass** die Antigene oder die entsprechenden "fördernden" Antikörper auf einem Träger des Chiptyps fixiert sind.

11. Satz oder Kit zum Ausführen eines diagnostischen In-vifro-Tests gemäß einem der Ansprüche 1 bis 10, der
- das interne strukturelle Virusprotein VP4 oder ein Fragment desselben oder die entsprechenden "fördernden" Antikörper,
- die Reagenzien zum Schaffen des zur Durchführung der Antigen-Antikörper-Reaktion günstigen Mediums und
- die Reagenzien, die den Nachweis des gebildeten Komplexes erlauben, umfasst.

12. Verwendung des diagnostischen Tests gemäß einem der Ansprüche 1 bis 10 zur Vorhersage der Entstehung einer chronischen humanen oder tierischen Erkrankung, die mit einer Infektion durch ein Enterovirus zusammenhängt.

13. Verwendung des diagnostischen Tests gemäß einem der Ansprüche 1 bis 10 zur Vorhersage der Entstehung eines Diabetes Typ 1 bei einem prädiabetischen Patienten.

14. Verwendung des diagnostischen Tests gemäß einem der Ansprüche 1 bis 10 zur Festlegung des Stadiums einer chronischen humanen oder tierischen Erkrankung, die mit einer Infektion durch ein Enterovirus zusammenhängt.

15. Verwendung des diagnostischen Tests gemäß einem der Ansprüche 1 bis 10 zur Festlegung des Stadiums eines Diabetes Typ 1, der mit einer Infektion durch CVB zusammenhängt.

16. Verwendung des diagnostischen Tests gemäß einem der Ansprüche 1 bis 10 zur Bestimmung des Serotyps des für eine akute humane oder tierische Infektion verantwortlichen Enterovirus.

17. Verwendung des diagnostischen Tests gemäß einem der Ansprüche 1 bis 10 zur Bestimmung der Verteilung von Enterovirusinfektionen in einer gegebenen humanen oder tierischen Population nach dem Serotyp.

18. Verwendung des diagnostischen Tests gemäß einem der Ansprüche 1 bis 10 zur In-vitro-Bestimmung des viralen Ziels eines antiviralen Mittels.
